# EUROPEAN PATENT SPECIFICATION

(11) **EP 1 607 110 B1**
(45) Date of publication and mention of the grant of the patent: **25.01.2012**
(21) Application number: 04722424.1
(22) Date of filing: 22.03.2004
(51) Int. Cl.: A61M 1/36

(54) **BLOOD FILTER DEVICE AND METHOD OF PRODUCING THE SAME**
BLUTFILTERVORRICHTUNG UND VERFAHREN ZU IHRER HERSTELLUNG
DISPOSITIF DE FILTRAGE DE SANG ET PROCEDE DE REALISATION

(30) Priority: 24.03.2003 JP 2003081092
(43) Date of publication of application: 21.12.2005
(73) Proprietor: JMS Co. Ltd., Hiroshima-shi, Hiroshima 730-8652 (JP)
(72) Inventor: KAWARABATA, Shigeki, . (JP); KATSUNO, Yutaka, . (JP); NAKAO, Shota, . (JP)
(74) Representative: Schwarzensteiner, Marie-Luise
(86) International application number: PCT/JP2004/003835
(87) International publication number: WO 2004/084974

(56) References cited:
- WO-A-96/33770
- GB-A- 1 440 027
- JP-A- 9 508 564
- JP-A- 2000 517 240
- JP-U- 4 106 605
- US-A- 3 827 562

## Description

### Technical Field

The present invention generally relates to a blood filter device used for filtering foreign substances, thrombi, and the like in an artificial heart-lung circuit. In particular, the present invention relates to a blood filter device configured so that air bubbles remaining in a filter can be removed easily and to a method of producing the same.

### Background Art

Nowadays, there has been a growing trend to incorporate a blood filter device such as an arterial filter in an artificial heart-lung circuit used for heart surgery involving extracorporeal circulation for the sake of safety. To provide adequate security for patients, it has been demanded strongly that such a blood filter device be configured so that it can remove minute foreign substances in the artificial heart-lung circuit, thrombi formed during an operation, or air that has entered or been released from the circuit so as not to allow them to enter the patient body.

A filter generally used in the blood filter device is a polyester screen filter with pores of about 20 to 40 µm that has been pleated and then formed into a cylindrical shape. For example, Japanese Patent No. 3270193 discloses that a sheet-like filter member is folded so as to have a plurality of pleats and the pleated filter member is then formed into a cylindrical shape in which the respective pleats are arranged radially with peaks thereof positioned on the outer circumference side and valleys thereof positioned on the inner circumference side. The thus-formed cylindrical filter is disposed in a cylindrical housing. In the filter configured as above, blood flows in the housing in the radial direction of the cylindrical filter member to pass therethrough, which allows dirt, impurities, thrombi, and the like contained in the blood to be removed effectively, as disclosed in Japanese Patent No. 3012692 and JP 2000-60967 A, for example.

In the filter as described above, blood first flows into an upper part of the cylindrical filter member, passes through the cylindrical filter member in its radial direction via an outer part of the filter member, and then flows out from a lower part of the cylindrical filter member via an inner part of the filter member. In this filter, the filter member surface extends vertically. This poses a problem in that, when a priming solution flows into the filter surface during a priming operation, air bubbles are liable to remain in the filter. Moreover, it is difficult to discharge the remaining air bubbles to the outside. This is because, since the filter member surface extends vertically, the air bubbles cannot be released from the filter easily, so that it takes quite a long time to remove the air bubbles completely.

More specifically, air bubbles remaining in the filter can be released with an impact from the outside caused by, for instance, flicking the housing with a finger. In this case, however, although the air bubbles can be released temporarily by giving an impact from a portion close to a position where the air bubbles adhere, they are liable to adhere again to an adjacent pleat of the filter. Thus, it is difficult to bring the air bubbles to an air vent provided above.

WO 96/33770 discloses a blood filter having a plurality of concentric annular pleats and a support element having a concentric annular shape.

### Disclosure of Invention

In order to solve the above-described problem, it is an object of the present invention to provide a blood filter device that can remove impurities, thrombi, and the like in blood effectively and also can discharge air bubbles remaining in a filter easily.

The present invention provides a blood filter device according to claim 1. Preferred embodiments are given in subclaims 2 to 13.

The present invention also provides a method of producing a blood filter device configured as above. The method is defined in claims 14 and 15.

### Brief Description of Drawings

FIG. 1A is a front view showing a blood filter device according to one embodiment of the present invention, FIG. 1B is a plan view of the same, and FIG. 1C is a cross-sectional view of the same.
FIG. 2 is a perspective view illustrating how blood flows in an upper part of the blood filter device.
FIG. 3A is a perspective view showing a schematic configuration of a filter retaining portion of the blood filter device, and FIG. 3B is a plan view of the same.
FIG. 4A is a cross-sectional view showing an upper half of a housing that constitutes the blood filter device, FIG. 4B is a bottom view of the same, and FIG. 4C is a cross-sectional view taken along line A-A of FIG. 4B.
FIG. 5A is a plan view showing a lower half of the housing, and FIG. 5B is a cross-sectional view of the same.
FIG. 6 is a partially cutaway perspective view showing a method of producing a blood filter device according to one embodiment of the present invention.
FIG. 7 is a plan view showing another configuration of the filter retaining portion.

### Description of the Invention

In the blood filter device according to the present invention, the filter is formed of a sheet-like filter member that has been folded so as to have a plurality of pleats with enveloping surfaces connecting top ends of the respective pleats being flat so that the filter as a whole has a plate-shaped outer shape, and the filter is disposed in the blood filter device so that the flat enveloping surfaces extend horizontally. With this configuration, air bubbles that remain on the filter surface during a priming operation can be removed easily by merely giving a physical impact to the housing, because there is no obstruction either above or below the filter.

The above-described blood filter device may be configured so that a space between an inner side wall of the filter retaining portion and an outer peripheral portion of the filter is filled with a resin so as to be sealed, and the filter is fixed to the inner side wall of the filter retaining portion with the resin. This allows the filter to be fixed reliably and also allows the space between the inner side wall of the filter retaining portion and the outer peripheral portion of the filter to be sealed reliably.

It is preferable that the ratio h/r of a height h of the dome portion to an inner diameter r of the dome portion on a filter retaining portion side is in the range from 0.26 to 1.06. More preferably, the ratio h/r is in the range from 0.44 to 0.91. Also, it is preferable that the ratio d/r of a depth d of the bottom portion to the inner diameter r of the dome portion on the filter retaining portion side is in the range from 0.11 to 0.30.

Also, it is preferable that the inner diameter r of the dome portion on the filter retaining portion side is 27 to 33 mm and the height h of the dome portion is 7 to 35 mm. With this configuration, it is possible to secure a sufficient air bubble-trap amount. More preferably, the height h of the dome portion is 12 to 30 mm. Still more preferably, the depth d of the bottom portion is 3 to 10 mm. With this configuration, it is possible to achieve a suitable balance between air bubble-removing performance and an amount of blood required to fill the blood filter device. Alternatively, a distance between adjacent pleats of the filter may be set to 1.6 to 3.7 mm, and a height of each pleat may be set to 5 to 30 mm. This allows the removal of air bubbles after a priming operation to be carried out easily.

The filter can be formed only of a filter member having a function of filtering a foreign substance. Furthermore, the filter retaining portion may have a cylindrical cavity whose cross section taken in a horizontal direction is circular. Still further, an outer peripheral length of an internal space of the dome portion is reduced toward the top of the dome portion. Preferably, an inner surface of the bottom portion has no recess or protrusion.

In the method of producing a blood filter device according to the present invention, potting is performed while applying a centrifugal force so that the space between the inner side wall of the retaining portion inner cylinder and the outer peripheral portion of the filter is sealed with the resin. This allows a plurality of effects, such that the pleats of the filter can be bonded to each other, the pleats of the filter can be supported by the filter retaining portion, etc., to be obtained at the same time. Therefore, according to the production method of the present invention, the blood filter device can be produced with an extremely efficient production process.

In this production method, it is preferable that holding ribs that extend vertically are provided at positions on the inner side wall of the filter retaining portion corresponding to end portions of the respective pleats, and when disposing the filter in the cavity of the filter retaining portion, the end portions of the pleats are inserted to the holding ribs, respectively, so that the filter is temporarily held by the inner side wall of the filter retaining portion.

Also, it is preferable that, for forming the housing, an upper half and a lower half are provided that are to be joined to each other so that a joint between the upper half and the lower half is in the filter retaining portion of the housing, the filter is disposed in a portion corresponding to the cavity of the filter retaining portion in one of the upper half and the lower half, and the other one of the upper half and the lower half is joined to the one of the upper half and the lower half, and thereafter, the sealing and the hardening of the resin are performed.

Hereinafter, a blood filter device according to the present invention will be described by way of an embodiment with reference to the drawings

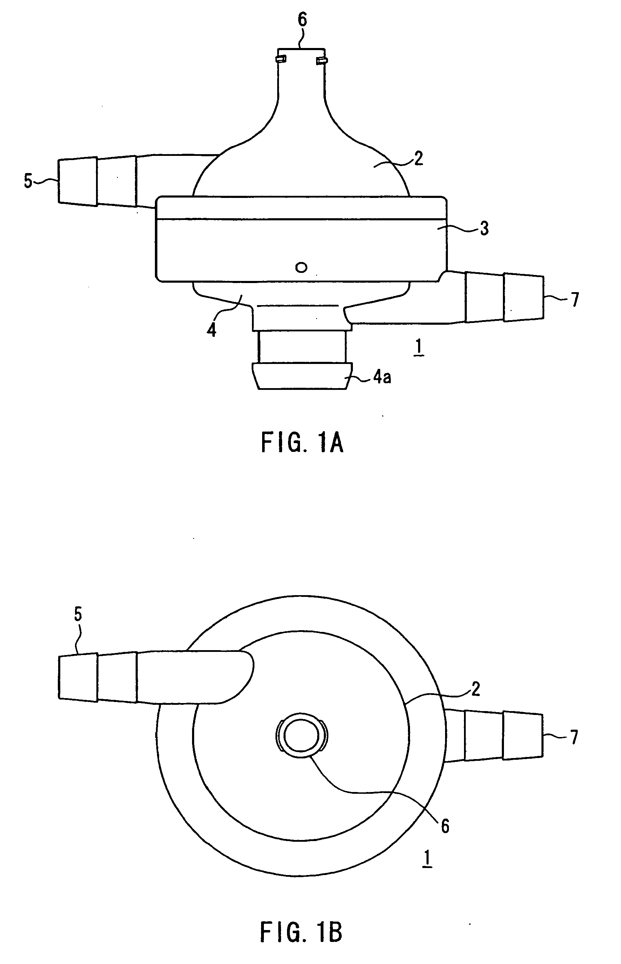

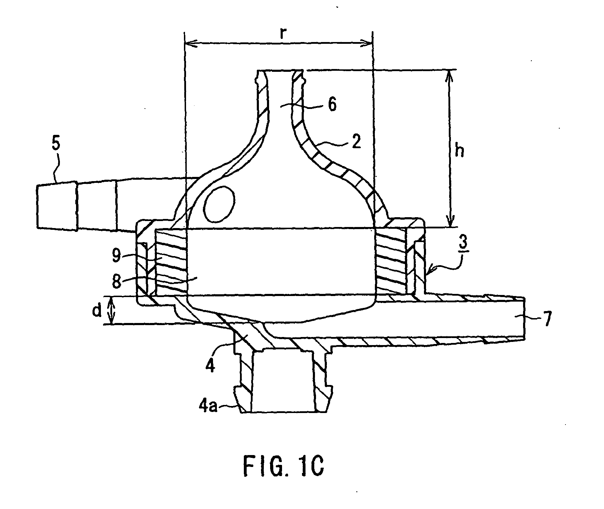

FIG. 1A is a front view of a blood filter device, FIG. 1B is a plan view of the same, and FIG. 1C is a cross-sectional view of the same. Reference numeral 1 denotes a housing that is made of resin, for example. The housing 1 includes a dome portion 2 forming an upper part of the housing, a filter retaining portion 3 forming a middle part of the housing, and a bottom portion 4 forming a lower part of the housing. The housing 1 has a horizontal cross section of a circular shape.

On a lateral portion of the dome portion 2, an inlet 5 is provided so as to allow blood to flow into the dome portion 2 horizontally and along an inner wall of the dome portion 2. On the top of the dome portion 2, an air vent 6 for discharging air such as air bubbles is provided. An outlet 7 for blood is provided in the bottom portion 4. The liquid that has flowed into the dome portion 2 from the inlet 5 passes through the filter retaining portion 3 and then flows out from the outlet 7. The bottom portion 4 also has a support portion 4a, which is used when installing the filter device and is irrelevant to the filtering function.

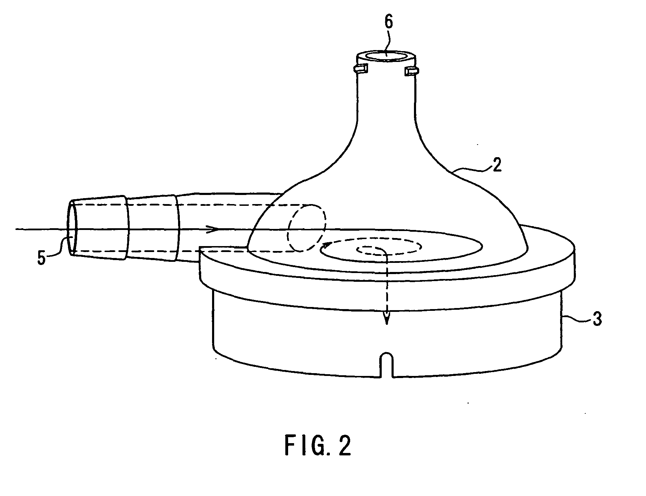

The dome portion 1 is formed so that an inner diameter thereof is reduced gradually toward the top of the dome portion 1. This allows air bubbles contained in blood to be released easily and the air bubbles thus released to move upward along the inner peripheral surface of the dome portion 1. Furthermore, since the dome portion 1 has a horizontal cross section of a circular shape and the inlet 5 is provided so as to allow blood to flow into the dome portion 2 horizontally and along an inner wall of the dome portion 2, the blood that has flowed into the dome portion 2 from the inlet 5 flows along the inner peripheral surface of the dome portion 2, thereby causing a swirling flow as indicated by the solid line in FIG. 2. The blood flow that has turned into a swirling flow gradually slows down. Thus, as indicated by the broken line in FIG. 2, a portion of the blood with a reduced velocity moves downward so that the blood gradually flows into the filter retaining portion 3. The shape of the dome portion 1 is not limited to that shown in FIG. 1A etc. as long as the dome portion 1 is formed so that an outer diameter thereof is reduced toward the air vent 6. For instance, the dome portion 1 may have a conical shape or a funnel shape.

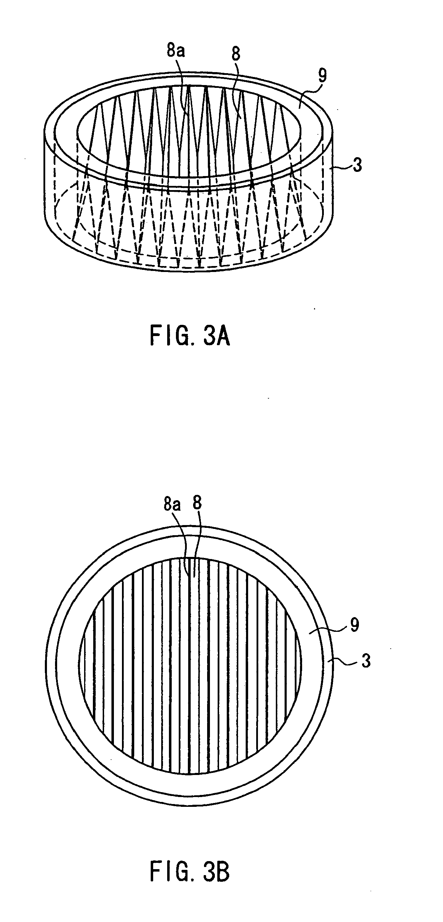

The filter retaining portion 3 has a cylindrical shape. As shown in FIG. 1C, a filter 8 for filtering foreign substances contained in blood is disposed in the filter retaining portion 3. As schematically shown in FIGs. 3A and 3B, the filter 8 is formed of a filter member that is a sheet-like mesh material folded so as to have a plurality of pleats 8a with enveloping surfaces connecting top ends of the respective pleats 8a being flat so that the filter 8 as a whole has a plate-shaped outer shape. The filter 8 partitions a cavity of the housing 1 into a dome portion 2 side and a bottom portion 4 side. The respective pleats 8a are aligned in parallel in the direction along a chord of the filter retaining portion 3. In FIG. 3B, thick solid lines indicate peaks of the pleats 8a, and thin solid lines indicate valleys of the pleats 8a. Note here that although FIGs. 3A and 3B show the filter retaining portion 3 as an independent cylindrical member for the sake of simplicity in illustration, the filter retaining portion 3 actually is formed continuously with the dome portion 2 or the bottom portion 4.

In the state where the filter 8 is disposed as shown in FIGs. 3A and 3B, a space between an inner side wall of the filter retaining portion 3 and an outer peripheral portion of the filter 8 is filled with a sealing resin 9, which may be made of, for instance, urethane resin, so as to be sealed, and the filter 8 is fixed to the inner side wall of the filter retaining portion 3 with the sealing resin 9. By disposing the filter 8 in the above-described manner, the blood with the reduced velocity that has flowed into the filter retaining portion 3 as shown in FIG. 2 can pass through the filter 8 without leaking out. As a result, only the blood subjected to the filtering flows into the bottom portion 4.

Furthermore, air bubbles that remain in the filter 8 when a priming solution passes through the filter 8 during a priming operation can be discharged easily from the air vent 6 at the top of the dome portion 2 or from the outlet 7 in the bottom portion 4 with an impact applied vertically to the filter 8 from the outside, for example, by flicking the bottom portion 4 with a finger. More specifically, because there is no obstruction either above or below the filter 8, air bubbles released from the filter 8 do not adhere to another portion of the filter 8 again, which ensures that they reach the air vent 6 at the top of the dome portion 2 or the outlet 7 in the bottom portion 4 and are discharged therefrom.

Furthermore, in the filter device according to the present embodiment, with the configuration in which the filter 8 as a whole has a plate-shaped outer shape with the enveloping surfaces connecting the top ends of the respective pleats being flat, the following effect also can be obtained. That is, since the filter 8 can maintain its shape well on its own, it is possible to form the filter 8 using only a mesh material as a filter member. In contrast, with a conventional configuration, it is necessary to use a support net in combination with the mesh material to maintain the shape of the filter 8. When the filter 8 is formed only of a mesh material without using a support net, air bubbles can be removed easily and besides, a loss of the blood flow pressure also can be reduced.

The bottom portion 4 provides a predetermined space under the filter 8. This allows a loss of the blood flow pressure passing through the filter device to be reduced to a negligible level in practical use. An inner surface of the bottom portion 4 is smooth and free from a projection or a recess. This allows the blood that has passed through the filter retaining portion 3 to be led to the outlet 7 without being impeded. Thus, the formation of thrombus or the like in the blood that has passed through the filter retaining portion 3 can be suppressed.

When the outlet 7 is provided in the lowest portion of the bottom portion 4 as shown in FIG. 1A, a portion where blood flow might be impeded is less liable to be formed. Furthermore, the outlet 7 may be formed so as to include a portion extending in the direction toward the center of the bottom portion 4 as shown in FIG. 1B. Alternatively, the outlet 7 may be formed so as to include a portion extending in the direction along the side face of the bottom portion 4.

As the filter member, it is possible to use a mesh material, a woven fabric, a non-woven fabric, or the like, or a combination of two or more of them, for example. The filter member can be made of polyester, polypropylene, polyamide, fluorocarbon fiber, stainless steel, or the like.

It is preferable that the housing 1, especially a horizontal cross section of the dome portion 2, has a circular shape because it is desired to cause a swirling flow of blood. However, it is to be noted here that other shapes such as an oval shape also can produce the same effect as described above. In the present embodiment, an inner diameter r (see FIG. 1C) of the dome portion 2 on the filter retaining portion 3 side is the same as that of the sealing resin 9 and that of the bottom portion 4 on the filter retaining portion 3 side, so that the inner surface of the housing 1 has no stepped portion at their boundaries.

In addition to the above-described effect, the blood filter device according to the present embodiment also is advantageous in that it can be made smaller than conventional blood filter devices while maintaining the filtering function satisfactory in practical use. However, to this end, it is desirable to set parameters with regard to the shape of the cavity of the housing 1 and the shape of the filter 8 as follows. The parameters to be set are as follows: an inner diameter r of the dome portion 2, a height h of the dome portion 2, and a depth d of the bottom portion 4, which are shown in FIG. 1C, and a ratio h/r of the height h of the dome portion 2 to the inner diameter r of the dome portion 2 and the ratio d/r of the depth d of the bottom portion 4 to the inner diameter r of the dome portion 2.

First, the ratio h/r preferably is in the range from 0.26 to 1.06. If the ratio h/r is smaller than 0.26, an angle between an inner wall surface of the dome portion 2 and the horizontal plane is too small, so that sufficient air bubble-removing performance cannot be attained. On the other hand, if the ratio h/r is greater than 0.61, an amount of liquid required to fill the dome portion 2 is too great. More preferably, the ratio h/r is in the range from 0.44 to 0.91.

The ratio d/r preferably is in the range from 0.11 to 0.30. If the ratio d/r is smaller than 0.11, an angle between an inner wall surface of the bottom portion 4 and the horizontal plane is too small, so that sufficient air bubble-removing performance cannot be attained. On the other hand, if the ratio d/r is greater than 0.30, an amount of liquid required to fill the bottom portion 4 is too great.

Furthermore, from the viewpoint of an air bubble-trap amount, the inner diameter r of the dome portion 2 may be set to 27 to 33 mm, and the height h of the dome portion 2 may be set to 7 to 35 mm. By setting the inner diameter r and the height h dome portion 2 within these ranges, it is possible to achieve an air bubble-trap amount of at least 5 mL at a blood flow rate of 1.5 L/min, as required in practical use. It is more preferable to set the height h to 12 to 30 mm. Note here that the "air bubble-trap amount" is defined herein as an amount of air bubbles blocked by the filter 8 to be captured and accumulated in the dome portion 2 when a liquid for measuring this amount is supplied to the filter device. The method of measuring the air bubble-trap amount will be described later. In the following, an advantageous effect on an air bubble-trap amount obtained by setting the parameters as described above will be described.

First, as a precondition for setting the above-described parameters, a desirable filter membrane area will be described. A generally used blood filter for infants is required to have a maximum blood flow rate of 1.5 L/min in practical use. In order to restrict a pressure loss at this blood flow rate to a negligible level in practical use, a total area of pores in a mesh material that serves as a filter member needs to be substantially 8 cm² or greater.

On the other hand, the mesh material generally has substantially uniform pores of 20 to 40 µm, and the porosity thereof desirably is 16% to 28%. When the porosity is less than 16%, the loss of the blood flow pressure is too great. On the other hand, when the porosity is more than 28%, the mesh material cannot remove a foreign substance, a thrombus, or the like that is 40 µm or larger, while such is a filtering function required in practical use. In order to allow the total area of the pores in the mesh material to be substantially 8 cm² or greater as described above while satisfying the above-described porosity range, an area of the mesh material, i.e., the filter membrane, needs to be 29 cm² to 50 cm². Considering the variation in working conditions of the filter device, these values are multiplied by a safety factor 1.5. Therefore, the mesh material (the filter membrane) needs to have an area of 44 cm² to 75 cm².

Parameters that allow the filter device of the present embodiment to achieve an air bubble-trap amount satisfactory in practical use when the filter membrane area falls within the above-described range were examined through experiments. As a result of experiments conducted to determine the inner diameter r and the height h of the dome portion 2 that allow the filter device to achieve an air bubble-trap amount of 5 mL or greater at blood flow rate of 1.5 L/min, it was found that the inner diameter r should be 27 to 33 mm and the height h should be 7 to 20 mm as described above.

The air bubble-trap amount was measured in the following manner. As a liquid for measuring the air bubble-trap amount, citrated bovine blood (37°C, Ht.: 35%, T.P.: 6 g/dL) was supplied to the filter device shown in FIG. 1. Ht. represents a hematocrit, and T.P. represents a total protein content in plasma. Before supplying the liquid to the filter device, air bubbles were injected into the liquid at an injection rate of 2 mL/min. While monitoring the air bubbles contained in the liquid that had flowed out from the outlet 7, the liquid supply to the filter device was maintained until the detection of an air bubble of 40 µm or larger. At the moment of the detection of an air bubble of 40 µm or larger, an amount (a volume at atmospheric pressure) of air bubbles accumulated in the dome portion 2 was measured, and the thus-obtained measured value was regarded as the air bubble-trap amount.

When the area of the filter membrane and the inner diameter r and the height h of the dome portion 2 satisfy the above-described ranges, the volume of the filter device of the present embodiment can be reduced to about half the volumes of conventional filter devices.

With regard to the parameters other than those described above, it is desirable that the depth d of the bottom portion 4 is in the range from 3 to 10 mm, from the aspect of the amount of blood required to fill the blood filter device. The reason for this is as follows. First, in order to allow easy discharge of air bubbles in blood flowing in the filter device from the outlet 7 of the bottom portion 4, it is necessary that the depth d is at least 3 mm. Furthermore, in order to make the amount of blood required to fill the blood filter device not greater than 15 mL as required when treating infants, it is necessary that the depth d is not greater than 10 mm.

Furthermore, as parameters with regard to the pleats of the filter 8, a distance between adjacent pleats and a height of each pleat may be set in particular ranges in order to allow air bubbles to be removed easily. It is desirable that the distance between adjacent pleats is 1.6 to 3.7 mm and the height of each pleat is 5 to 30 mm. When the distance between adjacent pleats is smaller than 1.6 mm, air bubbles cannot be removed easily. On the other hand, when the distance between adjacent pleats is greater than 3.7 mm, it is difficult to obtain a sufficient filter membrane area. When the height of each pleat is smaller than 5 mm, it is difficult to obtain a sufficient filter membrane area. On the other hand, when the height of each pleat is greater than 30 mm, the volume of the filter retaining portion 3 increases accordingly, which may result in an increase in the amount of blood required to fill the blood filter device.

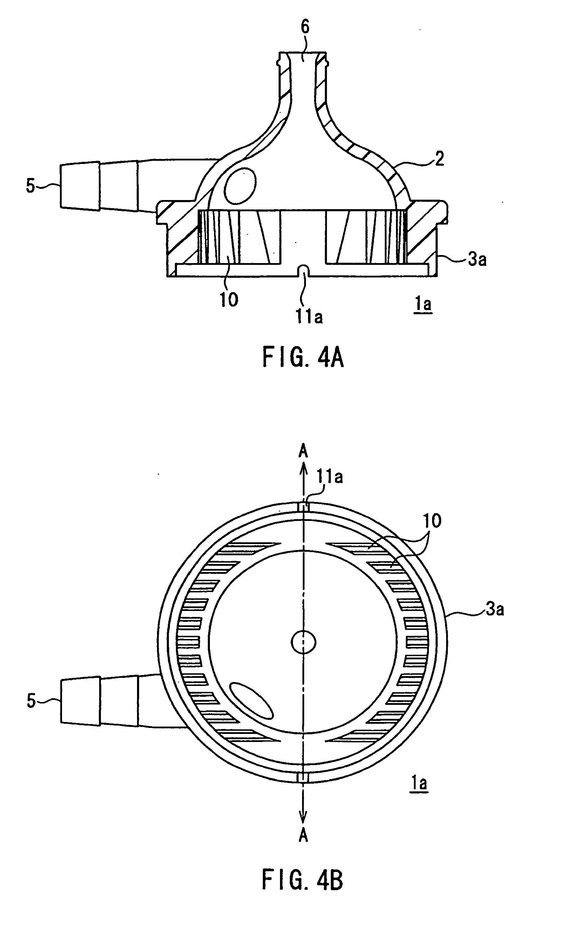

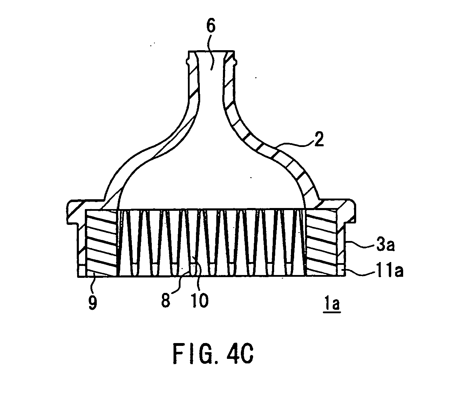

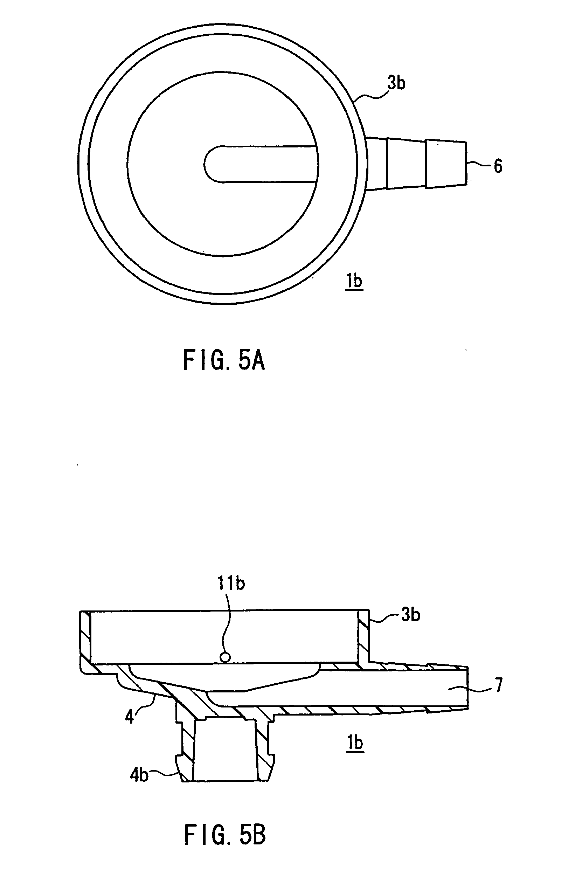

Next, a method of producing the filter device according to the present embodiment will be described with reference to FIGs. 4A to 4C, FIGs. 5A and 5B, and FIG. 6. FIG. 4A is a cross-sectional view showing an upper half 1a of the housing that constitutes the blood filter device, FIG. 4B is a bottom view of the same, and FIG. 4C is a cross-sectional view taken along line A-A of FIG. 4B. FIG. 5A is a cross-sectional view showing a lower half 1b of the housing, and FIG. 5B is a plan view of the same. Note here that only FIG. 4C shows the filter 8 and the sealing resin 9.

These drawings show basically the same configuration as described above, but holding ribs 10 for supporting the filter 8 temporarily also are shown in these drawings (see FIGs. 4A to 4C). In the upper half 1a and the lower half 1b of the housing, a retaining portion inner cylinder 3a and a retaining portion outer cylinder 3b for constituting the filter retaining portion 3 are formed, respectively. The upper half 1a and the lower half 1b are joined together by fitting the retaining portion inner cylinder 3a into the retaining portion outer cylinder 3b, thereby obtaining the housing as a single component.

As shown in FIGs. 4A to 4C, the holding ribs 10 are provided in the upper half 1a. The holding ribs 10 are disposed on an inner peripheral wall of the retaining portion inner cylinder 3a by forming grooves at portions of the inner peripheral wall corresponding to end portions of the respective pleats 8a (see FIG. 3A) of the filter 8. The grooves formed by the holding ribs 10 have a depth corresponding to a height of the holding ribs 10.

In the retaining portion inner cylinder 3a of the upper half 1a, a pair of notches 11a further is formed. In the retaining portion outer cylinder 3b of the lower half 1b, through holes 11b are formed at positions corresponding to the pair of notches 11a formed in the retaining portion inner cylinder 3a. When the upper half 1a is fitted into the lower half 1b, the notches 11a communicate with the through holes 11b, thereby forming holes that pass through peripheral walls of the retaining portion inner cylinder 3a and the retaining portion outer cylinder 3b. The reason for providing these holes will be described later.

In the production of the blood filter device, the upper half 1a and the lower half 1b of the housing and the filter 8 are formed first in the above described manner. Then, as shown in FIG. 4C, the filter 8 is disposed in the cavity of the retaining portion inner cylinder 3a of the upper half 1a of the housing so that the flat enveloping surfaces extend horizontally At this time, the end portions of the respective pleats of the filter 8 are inserted between the holding ribs 10 so that the filter 8 is temporarily held by the inner side wall of the retaining portion inner cylinder 3a.

Thereafter, the upper half 1a and the lower half 1b are joined together by fitting the retaining portion inner cylinder 3a into the retaining portion outer cylinder 3b, thereby obtaining the housing 1 as a single component.

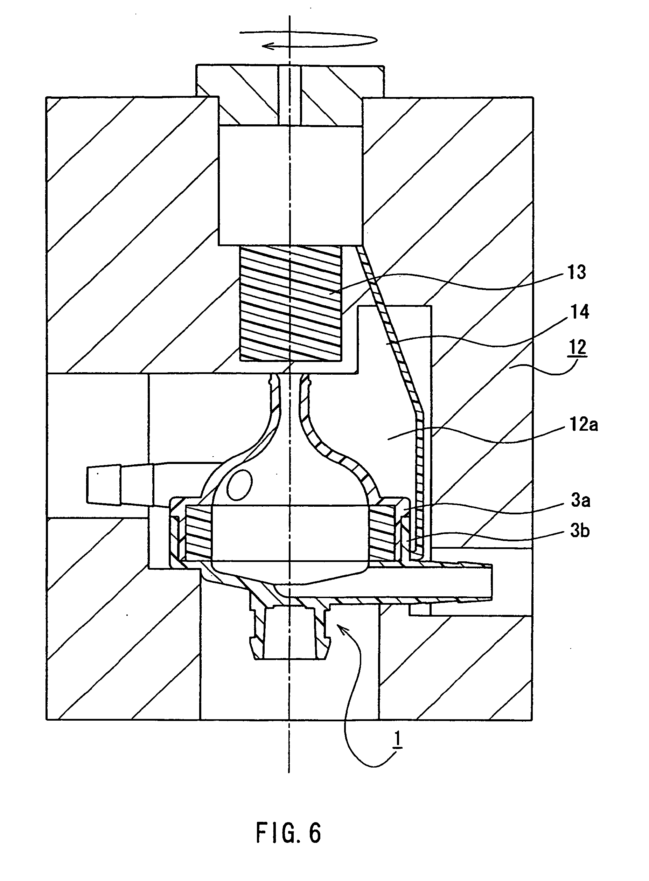

Next, as shown in FIG. 6, the housing 1 in which the filter 8 is disposed is set in a rotating jig 12. The rotating jig 12 has a cavity 12a with a predetermined shape for supporting the housing 1. When the rotating jig 12 is rotated, the housing 1 rotates together with the rotating jig 12. In an upper part of the rotating jig 12, a resin reservoir 13 containing a sealing resin such as urethane resin is provided, and a resin supply channel 14 extends from the resin reservoir 13 to a side face of the retaining portion outer cylinder 3b. The sealing resin supplied to the side face of the retaining portion outer cylinder 3b enters the cavity of the retaining portion inner cylinder 3b via the notch 11a and the through hole 11b (see FIGs. 4A to 4C and FIGs. 5A and 5B).

When the rotating jig 12 is rotated, the filter device is subjected to a centrifugal force that is caused by rotation around a center of the filter retaining portion 3a and acts horizontally. As a result, the sealing resin spills out of the resin reservoir 13 to be supplied to the retaining portion inner cylinder 3a through the resin supply channel 14, so that a space between an inner side wall of the retaining portion inner cylinder 3a and an outer peripheral portion of the filter 8 is filled with a resin. By hardening the resin filling the space, the filter 8 can be fixed to the inner side wall of the retaining portion inner cylinder 3a with the sealing resin 9, as shown in FIG. 1C.

When the space between the inner side wall of the retaining portion inner cylinder 3a and the outer peripheral portion of the filter 8 is sealed with the resin by performing potting while applying a centrifugal force as described above, the following six effects can be obtained at the same time:
(1) the shape of the pleats of the filter 8 is maintained;
(2) the pleats of the filter 8 are supported by the filter retaining portion 3;
(3) the holding ribs 10 are embedded in the resin;
(4) the upper half 1a and the lower half 1b of the housing are bonded to each other;
(5) the space between the inner side wall of the filter retaining portion 3 and the outer peripheral portion of the filter 8 is sealed; and
(6) the flow path is allowed to have a cross section such that there is no stepped portion at the boundary between the filter retaining portion 3 and the dome portion 2 or between the filter retaining portion 3 and the bottom portion 4.

Therefore, this production method can produce the filter device with an extremely simple process and thus is effective in reducing the production cost. Furthermore, the effect described in (3) contributes to the improvement of air bubble-removing performance. Moreover, by the effect described in (6), the inner wall surface of the housing can be made smooth, which contributes to the prevention of thrombus formation and to the improvement of air bubble-removing performance.

Note here that in the above-described production process, it is not always necessary to provide the holding ribs 10 in order to allow the filter 8 to be temporarily held by the inner side wall of the retaining portion inner cylinder. The filter 8 can be temporarily held by the inner side wall of the retaining portion inner cylinder 3a with other configurations.

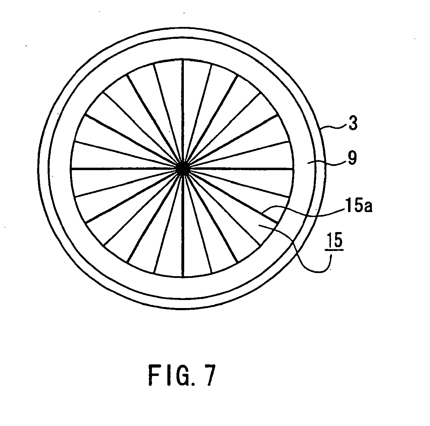

Moreover, the way of folding the filter 8 is not limited to that shown in FIGs. 3A and 3B in which the pleats 8a are aligned in parallel in the direction along a chord of the filter retaining portion 3, and may be, for example, as shown in FIG. 7. More specifically, in a filter 15 shown in FIG. 7, which does not fall under the scope of the present invention, pleats 15a are aligned so as to extend radially from the center of the filter retaining portion 3. Even when the pleats 12a are configured as above, the same effects as described above also can be obtained.

Although the filter 8 is folded so as to have a plurality of pleats, the present specification is not limited thereto. For example, the filter as shown in FIGs. 3A and 3B or FIG. 7 may be formed by folding a filter member in a wave-like form having peaks and valleys.

### Industrial Applicability

According to the blood filter device of the present invention, foreign substances, thrombi, etc. in blood can be removed reliably, and besides, air bubbles adhering to an upper surface of the filter can be removed easily with a physical impact caused by, for example, hitting the housing from above or below the housing.

## Claims

1. A blood filter device comprising:
a housing (1) that comprises a dome portion (2) forming an upper part of the housing (1), a filter retaining portion (3) with a cylindrical shape forming a middle part of the housing (1), and a bottom portion (4) forming a lower part of the housing (1);
an inlet (5) provided on a lateral portion of the dome portion (2) so as to allow blood to flow into the dome portion (2) horizontally and along an inner wall of the dome portion (2);
an air vent (6) provided at a top of the dome portion (2);
a filter (8) for filtering a foreign substance in the blood, the filter (8) being disposed in the filter retaining portion (3); and
an outlet (7) for the blood, the outlet (7) being provided in the bottom portion (4),
the blood filter device being configured so that the blood flows into the dome portion (2) from the inlet (5), passes through the filter retaining portion (3), and then flows out from the outlet (7),
wherein the filter (8) is formed of a sheet-like filter member that has been folded so as to have a plurality of pleats (8a) having a linear shape arranged in parallel with enveloping surfaces connecting top ends of the respective pleats (8a) being flat so that the filter (8) as a whole has a plate-shaped outer shape,
the filter (8) is arranged so as to partition a cavity of the housing (1) into a dome portion (2) side and a bottom portion (4) side, with the pleats (8a) extending across the filter retaining portion (3), and
a plurality of holding ribs (10) are provided on an inner peripheral wall of the filter retaining portion (3) at positions corresponding to end portions of the respective pleats (8a), and extending so as to form grooves whereby the end portions of the pleats (8a) are inserted between the holding ribs (10).

2. The blood filter device according to claim 1, wherein a space between an inner side wall of the filter retaining portion (3) and an outer peripheral portion of the filter (8) is filled with a resin (9) so as to be sealed, and the filter (8) is fixed to the inner side wall of the filter retaining portion (3) with the resin.

3. The blood filter device according to claim 1 or 2, wherein a ratio h/r of a height h of the dome portion (2) to an inner diameter r of the dome portion (2) on a filter retaining portion (3) side is in a range from 0.26 to 1.06.

4. The blood filter device according to claim 3, wherein the ratio h/r is in a range from 0.44 to 0.91.

5. The blood filter device according to claim 1 or 2, wherein a ratio d/r of a depth d of the bottom portion (4) to an inner diameter r of the bottom portion (4) on a filter retaining portion (3) side is in a range from 0.11 to 0.30.

6. The blood filter device according to claim 1 or 2, wherein an inner diameter r of the dome portion (2) on a filter retaining portion (3) side is 27 to 33 mm, and a height h of the dome portion (2) is 7 to 35 mm.

7. The blood filter device according to claim 6, wherein the height h of the dome portion (2) is 12 to 30 mm.

8. The blood filter device according to claim 6, wherein a depth d of the bottom portion (4) is 3 to 10 mm.

9. The blood filter device according to claim 6, wherein a distance between adjacent pleats (8a) of the filter (8) is 1.6 to 3.7 mm, and a height of each pleat (8a) is 5 to 30 mm.

10. The blood filter device according to any one of claims 1 to 9, wherein the filter (8) is formed only of a filter member having a function of filtering the foreign substance.

11. The blood filter device according to claim 1, wherein the filter retaining portion (3) has a cylindrical cavity whose cross section taken in a horizontal direction is circular.

12. The blood filter device according to claim 1, wherein an outer peripheral length of an internal space of the dome portion (2) is reduced toward the top of the dome portion (2).

13. The blood filter device according to claim 1, wherein an inner surface of the bottom portion (4) has no recess or protrusion.

14. A method for producing a blood filter device,
the blood filter device comprising:
a housing (1) that comprises a dome portion (2) forming an upper part of the housing (1), a filter retaining portion (3) with a cylindrical shape forming a middle part of the housing (1), and a bottom portion (4) forming a lower part of the housing (1);
an inlet (5) provided on a lateral portion of the dome portion (2) so as to allow blood to flow into the dome portion (2) horizontally and along an inner wall of the dome portion (2);
an air vent (6) provided at a top of the dome portion (2);
a filter (8) for filtering a foreign substance in the blood, the filter (8) being disposed in the filter retaining portion (3); and
an outlet (7) for the blood, the outlet (7) being provided in the bottom portion (4),
the blood filter device being configured so that the blood flows into the dome portion (2) from the inlet (5), passes through the filter retaining portion (3), and then flows out from the outlet (7),
the method comprising:
forming the filter (8) by folding a sheet-like filter member so as to have a plurality of pleats (8a) having a linear shape arranged in parallel with enveloping surfaces connecting top ends of the respective pleats (8a) being flat so that the filter (8) as a whole has a plate-shaped outer shape;
disposing the filter in a cavity of the filter retaining portion of the housing so that the flat enveloping surfaces extend horizontally so that the pleats (8a) extend across the filter retaining portion (3); and
filling a space between an inner side wall of the filter retaining portion (3) and an outer peripheral portion of the filter (8) with a resin (9) while applying a centrifugal force that is caused by rotation around a center of the filter retaining portion (3) and acts horizontally and then hardening the resin (9), thereby fixing the filter (8) to the inner side wall of the filter retaining portion (3) with the resin (9),
wherein holding ribs (10) are provided on an inner peripheral wall of the filter retaining portion (3) at positions corresponding to end portions of the respective pleats (8a) and extend so as to form grooves, and
when disposing the filter (8) in the cavity of the filter retaining portion (3), the end portions of the pleats (8a) are inserted between the holding ribs (10), respectively, so that the filter (8) is temporarily held by the inner side wall of the filter retaining portion (3).

15. The method according to claim 14, wherein, for forming the housing (1), an upper half (1a) and a lower half (1b) that are to be joined to each other so that a joint between the upper half (1a) and the lower half (1b) is in the filter retaining portion (3) of the housing (1) are provided,
the filter (8) is disposed in a portion corresponding to the cavity of the filter retaining portion (3) in one of the upper half (1a) and the lower half (1b), and the other one of the upper half (1a) and the lower half (1b) is joined to the one of the upper half (1a) and the lower half (1b), and thereafter,
the sealing and the hardening of the resin (9) are performed.

## Patentansprüche

1. Blutfiltervorrichtung, umfassend:
ein Gehäuse (1), das einen Domabschnitt (2), der einen oberen Teil des Gehäuses (1) bildet, einen Filterrückhalteabschnitt (3) mit zylindrischer Gestalt, der einen Mittelteil des Gehäuses (1) bildet, und einen Bodenabschnitt (4), welcher den unteren Teil des Gehäuses (1) bildet, umfasst,
einen Einlass (5), der auf einem Seitenabschnitt des Domabschnittes (2) vorgesehen ist, um Blut in den Domabschnitt (2) horizontal und entlang einer Innenwand des Domabschnittes (2) fließen zu lassen;
einen Entlüftungsstutzen (6), der oben am Domabschnitt (2) vorgesehen ist;
einen Filter (8), um eine Fremdsubstanz in dem Blut herauszufiltern, wobei der Filter (8) in dem Filterrückhalteabschnitt (3) angeordnet ist; und
einen Auslass (7) für das Blut, der am Bodenabschnitt (4) vorgesehen ist,
wobei die Blutfiltervorrichtung so konfiguriert ist, dass das Blut vom Einlass (5) in den Domabschnitt (2) fließt, den Filterrückhalteabschnitt (3) passiert und dann aus dem Auslass (7) fließt,
worin der Filter (8) aus einem blattartigen Filterelement gebildet ist, das so gefaltet wurde, dass es eine Vielzahl von Falten in linearer Form besitzt, die parallel mit Umhüllungsoberflächen angeordnet sind, welche obere flache Enden der jeweiligen Falten (8a) verbinden, so dass der Filter (8) als Ganzes eine lamellenförmige Außenkontur aufweist,
der Filter so angeordnet ist, dass er einen Hohlraum des Gehäuses (1) in eine Domabschnitt (2)-Seite und eine Bodenabschnitt (4)-Seite teilt, wobei die Falten (8a) sich über den Filterrückhalteabschnitt (3) erstrecken, und
eine Vielzahl von Halterippen (10) an einer Innenumfangswand des Filterrückhalteabschnittes (3) an Positionen vorgesehen sind, welche Endabschnitten der jeweiligen Falten (8a) entsprechen und sich so erstrecken, dass Riefen gebildet werden, wobei die Endabschnitte der Falten (8a) zwischen die Halterippen (10) eingeführt sind.

2. Blutfiltervorrichtung gemäß Anspruch 1, worin ein Abstand zwischen einer Innenseitenwand des Filterrückhalteabschnittes (2) und einer einem äußeren Umfangsabschnitt des Filtiers (8) mit einem Harz (9) dichtend gefüllt ist und der Filter (8) an die Innenseitenwand des Filterrückhalteabschnittes (3) mit dem Harz fixiert ist.

3. Blutfiltervorrichtung gemäß Anspruch 1 oder 2, worin ein Verhältnis h/r einer Höhe h des Domabschnittes (2) zu einem Innendurchmesser r des Domabschnittes (2) auf einer Filterrückhalteabschnitt (3)-Seite im Bereich von 0,26 bis 1,06 liegt.

4. Blutfiltervorrichtung gemäß Anspruch 3, worin das Verhältnis h/r im Bereich von 0,44 bis 0,91 liegt.

5. Blutfiltervorrichtung gemäß Anspruch 1 oder 2, worin ein Verhältnis d/r einer Tiefe d des Bodenabschnittes (4) zu einem Innendurchmesser r des Bodenabschnittes (4) auf einer Filterrückhalteabschnitt (3)-Seite in einem Bereich von 0,11 bis 0,30 liegt.

6. Blutfiltervorrichtung gemäß Anspruch 1 oder 2, worin ein Innendurchmesser r des Domabschnittes (2) auf einer Filterrückhalteabschnitt (3)-Seite 27 bis 33 mm beträgt und eine Höhe h des Domabschnittes (2) 7 bis 35 mm ist.

7. Blutfiltervorrichtung gemäß Anspruch 6, worin die Höhe h des Domabschnittes (2) 12 bis 30 mm beträgt.

8. Blutfiltervorrichtung gemäß Anspruch 6, worin eine Tiefe d des Bodenabschnittes (4) 3 bis 10 mm beträgt.

9. Blutfiltervorrichtung gemäß Anspruch 6, worin ein Abstand zwischen aneinandergrenzenden Falten (8a) des Filters (8) 1,6 bis 3,7 mm beträgt und eine Höhe jeder Falte (8a) 5 bis 30 mm ist.

10. Blutfiltervorrichtung gemäß einem jeden der Ansprüche 1 bis 9, worin der Filter (8) nur aus einem Filterelement gebildet ist, das die Funktion des Filterns von Fremdsubstanzen besitzt.

11. Blutfiltervorrichtung gemäß Anspruch 1, worin der Filterrückhalteabschnitt (3) eine zylindrische Ausnehmung aufweist, deren Querschnitt in horizontaler Richtung kreisförmig ist.

12. Blutfiltervorrichtung gemäß Anspruch 1, worin eine äußere Umfangslänge eines Innenraumes des Domabschnittes (2) zur Spitze des Domabschnittes (2) reduziert ist.

13. Blutfiltervorrichtung gemäß Anspruch 1, worin eine Innenoberfläche des Bodenabschnittes (4) weder Ausnehmungen noch Vorsprünge besitzt.

14. Verfahren zur Herstellung einer Blutfiltervorrichtung, wobei die Blutfiltervorrichtung umfasst:
ein Gehäuse (1), das einen Domabschnitt (2), der einen oberen Teil des Gehäuses (1) bildet, einen Filterrückhalteabschnitt (3) mit zylindrischer Gestalt, der einen Mittelteil des Gehäuses (1) bildet, und einen Bodenabschnitt (4), welcher den unteren Teil des Gehäuses (1) bildet, umfasst,
einen Einlass (5), der auf einem Seitenabschnitt des Domabschnittes (2) vorgesehen ist, um Blut in den Domabschnitt (2) horizontal und entlang einer Innenwand des Domabschnittes (2) fließen zu lassen;
umfasst einen Einlass (5), der auf einem Seitenabschnitt des Domabschnittes (2) vorgesehen ist, um Blut in den Domabschnitt (2) horizontal und entlang einer Innenwand des Domabschnittes (2) fließen zu lassen;
einen Entlüftungsstutzen (6), der oben am Domabschnitt (2) vorgesehen ist;
einen Filter (8), um eine Fremdsubstanz in dem Blut herauszufiltern, wobei der Filter (8) in dem Filterrückhalteabschnitt (3) angeordnet ist; und
einen Auslass (7) für das Blut, der am Bodenabschnitt (4) vorgesehen ist,
wobei die Blutfiltervorrichtung so konfiguriert ist, dass das Blut vom Einlass (5) in den Domabschnitt (2) fließt, den Filterrückhalteabschnitt (3) passiert und dann aus dem Auslass (7) fließt,
wobei das Verfahren umfasst :
Ausbilden des Filters (8) durch Falten eines blattförmigen Filterelementes zu einer Vielzahl von linear geformten Falten (8a), die parallel mit Umhüllungsoberflächen angeordnet sind, die mit oberen flachen Enden der jeweiligen Falten (8), verbunden sind, so dass der Filter (8) als Ganzes eine lamellenförmige äußere Gestalt besitzt;
Anordnen des Filters in einem Hohlraum des Filterrückhalteabschnittes des Gehäuses, so dass die flachen Umhüllungsoberflächen sich horizontal erstrecken, so dass die Falten (8) sich über den Filterrückhalteabschnitt (3) erstrecken; und
Ausfüllen eines Zwischenraumes zwischen einer Innenseitenwand des Filterrückhalteabschnittes (3) und einem äußeren Umfangsabschnitt des Filters (8) mit einem Harz (9), während eine Zentrifugalkraft angewendet wird, welche durch Rotation um ein Zentrum des Filterrückhalteabschnittes (3) verursacht wird und horizontal wirkt und dann Aushärten des Harzes (9), wodurch der Filter (8) an die Innenseitenwand des Filterrückhalteabschnittes (3) mit dem Harz (9) fixiert wird,
worin Halterippen (10) an einer Innenumfangswand des Filterrückhalteabschnittes (3) an Positionen vorgesehen sind, die Endabschnitten der jeweiligen Falten (8a) entsprechen und sich unter Ausbildung von Riefen erstrecken, und
beim Anordnen des Filters (8) in dem Hohlraum des Filterrückhalteabschnittes (3) die Endabschnitte der Falten (8) zwischen die Halterippen (10) eingeführt werden, so dass der Filter (8) temporär durch die Innenseitenwand des Filterrückhalteabschnittes (3) gehalten wird.

15. Verfahren gemäß Anspruch 14, worin zur Ausbildung des Gehäuses (1) eine obere Hälfte (1a) und eine untere Hälfte (1 b) vorgesehen sind, welche miteinander zu verbinden sind, so dass eine Verbindung zwischen der oberen Hälfte (1a) und der unteren Hälfte (1b) im Filterrückhalteabschnitt (3) des Gehäuses (1) liegt,
der Filter (8) in einem Abschnitt angeordnet wird, der dem Hohlraum des Filterrückhalteabschnittes (3) entweder in der oberen Hälfte (1a) oder in der unteren Hälfte (1b) entspricht und entweder die obere Hälfte (1a) oder die untere Hälfte (1b) mit entweder der oberen Hälfte (1a) oder der unteren Hälfte (1b) verbunden wird und danach das Abdichten und das Aushärten des Harzes (9) durchgeführt wird.

## Revendications

1. Dispositif de filtrage de sang, comprenant:
un boîtier (1), comprenant une partie de dôme (2), formant une partie supérieure du boîtier (1), une partie de retenue de filtre (3), ayant une forme cylindrique formant une partie médiane du boîtier (1), et une partie de fond (4) formant une partie inférieure du boîtier (1) ;
une entrée (5), prévue sur une partie latérale de la partie de dôme (2), de manière à permettre à du sang de s'écouler dans la partie de dôme (2), horizontalement et le long d'une paroi intérieure de la partie de dôme (2) ;
un évent d'air (6), prévu au-dessus de la partie de dôme (2) ;
un filtre (8), pour filtrer des substances étrangères présentes dans le sang, le filtre (8) étant disposé dans la partie de retenue de filtre (3) ; et
une sortie (7) pour le sang, la sortie (7) étant prévue dans la partie de fond (4),
le dispositif de filtrage de sang étant configuré de manière que le sang s'écoule dans la partie de dôme (2) à partir de l'entrée (5), passe par la partie de retenue de filtre (3) et, ensuite, s'écoule à l'extérieur, en partant de la sortie (7),
dans lequel le filtre (8) est formé d'un organe filtrant analogue à une feuille, ayant été plié de manière à présenter une pluralité de plis (8a) ayant une forme linéaire, agencés en parallèle avec des surfaces d'enveloppement connectant des extrémités supérieures des plis (8a) respectifs d'allure plate, de manière que le filtre (8) présente globalement une forme extérieure en plaque,
le filtre (8) est agencé de manière à subdiviser une cavité du boîtier (1) en un côté de partie de dôme (2) et un côté de partie de fond (4), les plis (8a) s'étendant en travers de la partie de retenue de filtre (3), et
une pluralité de nervures de maintien (10) sont prévues sur une paroi périphérique intérieure de la partie de retenue de filtre (3), en des positions correspondant à des parties d'extrémité des plis (8a) respectifs, et s'étendant de manière à former des gorges, de manière que les parties d'extrémité des plis (8a) soient insérées entre les nervures de maintien (10) .

2. Dispositif de filtrage de sang selon la revendication 1, dans lequel un espace, entre une paroi de côté intérieur de la partie de retenue de filtre (3) et une partie périphérique extérieure du filtre (8), est rempli d'une résine (9) de manière à être fermé de manière étanche, et le filtre (8) est fixé à la paroi de côté intérieur de la partie de retenue de filtre (3) avec la résine.

3. Dispositif de filtrage de sang selon la revendication 1 ou 2, dans lequel un rapport h/r, d'une hauteur h de la partie de dôme (2) et un diamètre intérieur r de la partie de dôme (2) sur un côté de la partie de retenue de filtre (3), est compris entre 0,26 et 1,06.

4. Dispositif de filtrage de sang selon la revendication 3, dans lequel le rapport h/r est compris entre 0,44 et 0,91.

5. Dispositif de filtrage de sang selon la revendication 1 ou 2, dans lequel un rapport d/r, d'une profondeur d de la partie de fond (4) et un diamètre intérieur r de la partie de fond (4) sur un côté de la partie de retenue de filtre (3), est compris entre 0,11 et 0,30.

6. Dispositif de filtrage de sang selon la revendication 1 ou 2, dans lequel un diamètre intérieur r de la partie de dôme (2) sur un côté de la partie de retenue de filtre (3) est compris entre 27 et 33 mm, et une hauteur h de la partie de dôme (2) est compris entre 7 et 35 mm.

7. Dispositif de filtrage de sang selon la revendication 6, dans lequel la hauteur h de la partie de dôme (2) est compris entre 12 et 30 mm.

8. Dispositif de filtrage de sang selon la revendication 6, dans lequel une profondeur d de la partie de fond (4) est compris entre 3 et 10 mm.

9. Dispositif de filtrage de sang selon la revendication 6, dans lequel une distance entre des plis (8a) adjacents du filtre (8) est compris entre 1,6 et 3,7 mm, et une hauteur de chaque pli (8a) est compris entre 5 et 30 mm.

10. Dispositif de filtrage de sang selon l'une quelconque des revendications 1 à 9, dans lequel le filtre (8) est formé seulement d'un organe filtrant ayant une fonction de filtrage des substances étrangères.

11. Dispositif de filtrage de sang selon la revendication 1, dans lequel la partie de retenue de filtre (3) présente une cavité cylindrique, dont la section transversale, observée en une direction horizontale, est circulaire.

12. Dispositif de filtrage de sang selon la revendication 1, dans lequel une longueur périphérique extérieure d'un espace interne de la partie de dôme (2) est réduite en évoluant vers le dessus de la partie de dôme (2).

13. Dispositif de filtrage de sang selon la revendication 1, dans lequel une surface intérieure de la partie de fond (4) ne présente ni cavité ni saillie.

14. Procédé pour produire un dispositif de filtrage de sang, le dispositif de filtrage de sang comprenant :
un boîtier (1), comprenant une partie de dôme (2), formant une partie supérieure du boîtier (1), une partie de retenue de filtre (3), ayant une forme cylindrique, formant une partie médiane du boîtier (1), et une partie de fond (4), formant une partie inférieure du boîtier (1) ;
une entrée (5), prévue sur une partie latérale de la partie de dôme (2), de manière à permettre à du sang de s'écouler dans la partie de dôme (2), horizontalement et le long d'une paroi intérieure de la partie de dôme (2) ;
un évent d'air (6), prévu au-dessus de la partie de dôme (2) ;
un filtre (8), pour filtrer des substances étrangères présentes dans le sang, le filtre (8) étant disposé dans la partie de retenue de filtre (3) ; et
une sortie (7) pour le sang, la sortie (7) étant prévue dans la partie de fond (4),
le dispositif de filtrage de sang étant configuré de manière que le sang s'écoule dans la partie de dôme (2) à partir de l'entrée (5), passe par la partie de retenue de filtre (3) et, ensuite, s'écoule à l'extérieur, en partant de la sortie (7),
le procédé comprenant :
le formage du filtre (8) par pliage d'un organe filtrant analogue à une feuille, de manière à présenter une pluralité de plis (8a) ayant une forme linéaire, agencés en parallèle avec des surfaces d'enveloppement connectant des extrémités supérieures des plis (8a) respectifs d'allure plate, de manière que le filtre (8) présente globalement une forme extérieure en plaque,
la disposition du filtre dans une cavité de la partie de retenue de filtre (3) du boîtier, de manière que les surfaces d'enveloppement plates s'étendent horizontalement, afin que les plis (8a) s'étendent en travers de la partie de retenue de filtre (3), et
le remplissage d'un espace, entre une paroi latérale intérieure de la partie de retenue de filtre (3) et une partie périphérique extérieure du filtre (8) avec une résine (9), tout en appliquant une force centrifuge provoquée par une rotation effectuée autour d'un centre de la partie de retenue de filtre (3) et agissant horizontalement et, ensuite, durcissement de la résine (9), de manière à fixer le filtre (8) à la paroi latérale intérieure de la partie de retenue de filtre (3) avec la résine (9),
dans lequel des nervures de maintien (10) sont prévues sur une paroi périphérique intérieure de la partie de retenue de filtre (3), en des positions correspondant à des parties d'extrémité des plis (8a) respectifs, et s'étendent de manière à former des gorges, et
lors de la disposition du filtre (8) dans la cavité de la partie de retenue de filtre (3), les parties d'extrémité des plis (8a) sont insérées entre les nervures de maintien (10), respectivement, de manière que le filtre (8) soit temporairement maintenu par la paroi latérale intérieure de la partie de retenue de filtre (3).

15. Procédé selon la revendication 14, dans lequel, pour former le boîtier (1), une moitié supérieure (1a) et une moitié inférieure (1b) devant être jointes l'une à l'autre, de manière qu'un joint, entre la moitié supérieure (1a) et la moitié inférieure (1b) soit situé dans la partie de retenue de filtre (3), sont prévues,
le filtre (8) est disposé dans une partie correspondant à la cavité de la partie de retenue de filtre (3), dans l'une de la moitié supérieure (1a) et la moitié inférieure (1b), et l'autre, de la moitié supérieure (1a) et la moitié inférieure (1b), est jointe à la première, de la moitié supérieure (1a) et la moitié inférieure (1b) et, ensuite,
le scellage et le durcissement de la résine (9) sont effectués.
